# EUROPEAN PATENT SPECIFICATION

(11) **EP 3 170 391 B1**
(45) Date of publication and mention of the grant of the patent: **06.03.2019**
(21) Application number: 14897730.9
(22) Date of filing: 30.09.2014
(51) Int. Cl.: A01N 1/02, A61K 31/047, A61K 31/728, A61K 31/737, A61K 9/00

(54) **LAMELLAR CORNEA PRESERVING SOLUTION**
KONSERVIERUNGSLÖSUNG FÜR LAMELLARE HORNHAUT
SOLUTION DE CONSERVATION DE LA CORNÉE LAMELLAIRE

(30) Priority: 18.07.2014 CN 201410344982
(43) Date of publication of application: 24.05.2017
(73) Proprietor: YOUVISION BIOTECH CO., LTD., High-tech Industrial Development Zone Guangzhou Guangdong 510663 (CN)
(72) Inventor: CHEN, Wei, Wenzhou Zhejiang 325000 (CN); ZHENG, Qinxiang, Wenzhou Zhejiang 325000 (CN); YANG, Jun, Wenzhou Zhejiang 325000 (CN); LIN, Yongliang, Wenzhou Zhejiang 325000 (CN)
(74) Representative: Appleyard Lees IP LLP
(86) International application number: PCT/CN2014/087955
(87) International publication number: WO 2016/008220

(56) References cited:
- EP-A1- 0 516 901
- WO-A1-97/37537
- CN-A- 1 631 138
- CN-A- 103 550 826
- US-A1- 2006 079 482
- SHEN, JIKUI ET AL.: 'A New Type Mid-term Cornea Preservation Liquid' FOREIGN MEDICAL SCIENCES (SECTION OF OPHTHALMOLOGY vol. 18, no. 6, 31 December 1994, pages 368 AND 370 - 371, XP008184151
- HU , QIYI ET AL.: 'Progress in Preservation of Cornea' INTERNATIONAL EYE SCIENCE vol. 3, no. 2, 30 June 2003, page 66, XP008184153

## Description

### Technical field

The present disclosure relates to the field of preservation of medical materials, particularly to a lamellar cornea preservation solution.

### Background

Corneal blindness caused by loss of corneal transparency is a major cause of vision loss, only secondary to cataract. Corneal graft transplant can replace turbid or diseased corneal tissues and recover the original structure and transparency of the cornea, which can achieve the purpose of protecting eye structure and vision recovery. Current types of corneal transplant are categorized into penetrating keratoplasty, lamellar keratoplasty and endothelial keratoplasty, according to graft components and surgical approaches. The operation number of the two latter, component transplants, increases year by year for their advantages of lower rejection rates and fewer long term complications, due to the reservation of part of the structure of the recipient cornea (endothelium layer or stroma appraoching the enthelium). In the United States, the penetrating keratoplasty accounts for about 50% of the total, while the proportion of lamellar keratoplasty is increasing every year. The lamellar keratoplasty accounts for more than 40% of the total corneal transplants in China, about 25% in Singapore, 11% in the United Kingdom, and the rate is increasing year by year. The lamellar keratoplasty, which is not limited by preservation time of the material, is especially suitable for countries and regions which is short of corneal donation, such as China, Asia, Europe and other countries.

The previous cornea preservation solutions, aiming to maintain the activity of endothelial cells, are suitable for the corneal grafts used in penetrating keratoplasty and/or the endothelial keratoplasty. However, the preservation solution for lamellar cornea material should be more concerned about conserving the corneal collagen fibers which is the main component of the lamellar material. In addition, the xeno transplants corneal materials need to be virus inactivated and sterilizated by irradiation. The sterilization treatment after virus inactivation for xeno transplants corneal materials aims to improve safety and modify biological properties, and this process should also protect the corneal collagen fibers from damage. So it is necessary to develop a new lamellar cornea preservation solution, which is capable of maintaining the original structure of the corneal collagen fibers as well as its transparency, also protecting it from destruction of irridation.

### Summary of the disclosure

The purpose of the present disclosure is to provide a lamellar cornea preservation solution, which overcome the disadvantages and drawbacks existed in prior arts. The lamellar cornea preservation solution is suitable for preserving lamellar cornea materials, which can maintain original structure of the corneal collagen fibers and corneal transparency, also prevent the corneal collagen fibers from being damaged due to irradiation and sterilization treatments.

In order to achieve the above purpose, the solution of present disclosure includes the following components:
200-500g/L of glycerol, 5-50g/L of hyaluronic acid, 10-25g/L chondroitin sulfate, 1-10g/L of an antioxidant, and 10-40mmol/L of a buffer salt solution, the pH value being 6.0-8.0.

The antioxidant in the preservation solution may be N-acetylcysteine or vitamin C. And the buffer salt solution may be phosphate buffer solution, carbonate buffer solution or Hepes buffer solution. The main features of the present cornea preservation solution are as follows:
The first feature is the use of glycerol which is a cryoprotectant. Glycerol protects the corneal collagen fibers from being damaged in freezing and thawing processes, and glycerol is a small molecular, non-cytotoxic, soluble in water and easy to be eluted. It has been verified that glycerol of a certain concentration can effectively keep corneal fibles dehydrated, prevent the collagen fibers from swelling and structure damage, and maintain the corneal thickness at a normal physiological level. Glycerol has functions of anti-bacteria and virus inactivation, which can suppress the growth of bacteria without the use of antibiotics.

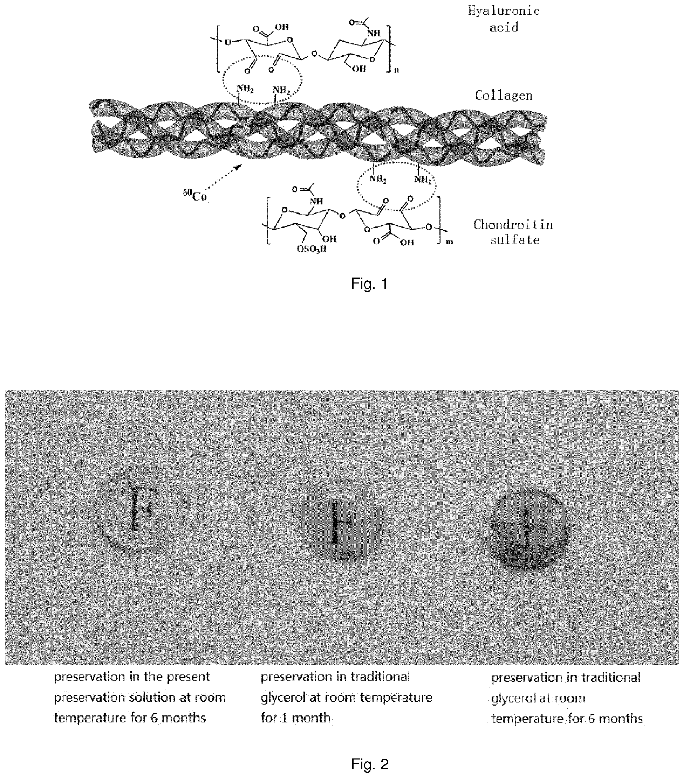

The second feature is the application of hyaluronic acid and chondroitin sulfate. Hyaluronic acid, a physiologically active substance, widely exists in animals and human. It has a high concentration in the human skin, aqueous humor and eye vitreous body. The hyaluronic acid liquid is of high viscosity and elasticity, and is widely used as an ophthalmic surgery adjuvant with excellent safety. Sodium hyaluronate is a hyaluronic acid with disaccharide units comprised of D-Glucuronic acid and N-acetylglucosamine, and its liquid can form a tertiary structure when the concentration of hyaluronic acid is more than 1%. Hyaluronic acid of an appropriate concentration also has dehydration effect, which can be used to maintain the thickness of the corneal collagen with glycerol. Most importantly, hyaluronic acid and chondroitin sulfate can bond to the breaking points of corneal collagen fibers, which can happen in th eprocess of γ -ray irradiation since the irridation can destruct the collagen fibers and cause collagen fibers aging and xanthochroia in some circumstance. The amino groups of hyaluronic acid and chondroitin sulfate can combine to the O bonds at a cleavage site to repair the structural integrity of the collagen fibers (FIG. 1). Before irradiation, sodium hyaluronate is of good viscoelasticity and can help maintain the natural shape of the cornea, the natural corneal curvature as well as the conformation of the collagen fibers during irradiation, so as to avoid corneal deformation caused by a certain degree of cross-linking due to irradiation. After irradiation, macromolecular hyaluronic acid is decomposed into micromolecular sodium hyaluronate, making the preservation solution be a liquid with a certain degree of viscosity which is very convenient for clinical surgery.

The third feature is the use of antioxidants. N-acetylcysteine (NAC), a strong free radical scavenger, can effectively prevent oxidative damage. Damages of collagen fibers during the irradiation or freezing and thawing processes can produce over-production of free radicals and cause oxidative damage to the collagen, then accelerating collagen aging and xanthochroia. The application of NAC effectively remove the free radicals and prevent the oxidative damage to the corneal colllegen.

The forth feature is the simple preparation of the preservation solution. The technology of the present preservation solution is not difficult and can be widely applied, not only in preserving the cornea grafts, but also for artificial skin and other collagen tissues.

The function mechanism and preservation results of the present disclosure are shown in Figures 1-2.

The present disclosure will be further described as below, with figures, legends and specific production methods.

### Brief description of the drawings

FIG. 1 The mechanism diagram of the present disclosure.
FIG. 2 The comparison of preservation results between the present disclosure and the traditional glycerol.

### Detailed description of preferred embodiments

The following examples of the present disclosure will be specifically described, which is an example of the present disclosure but not a limitation. A engineer in this field can make some non-essential improvements and adjustments to the present disclosure.

Formulation examples of the lamellar cornea preservation solution of the present disclosure:

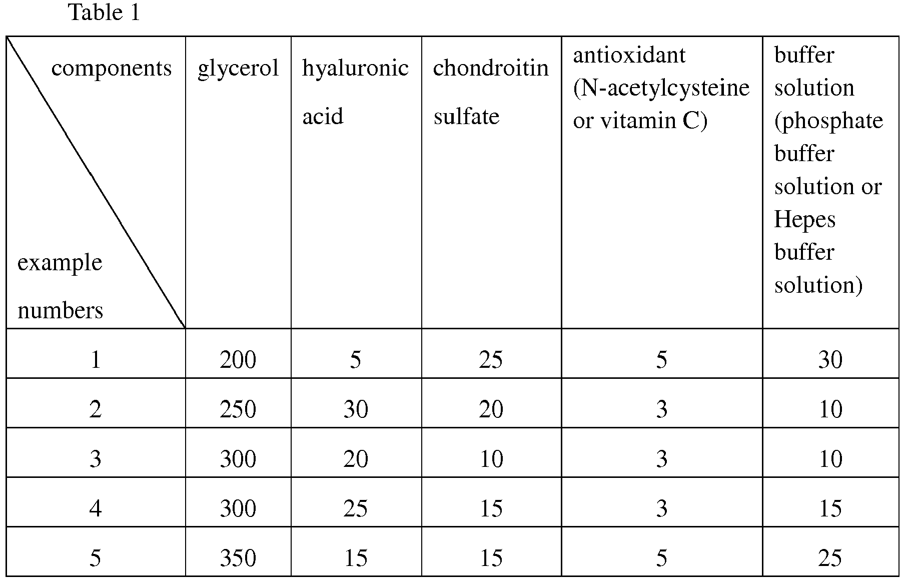

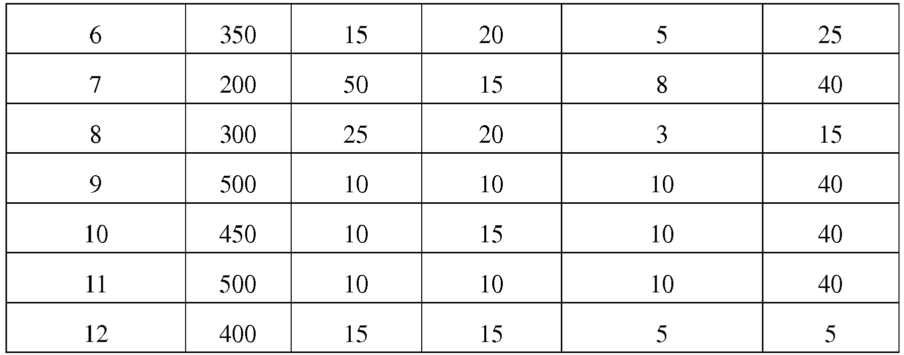

The corneal preservation solution prepared by the present disclosure can improve the preservation and transparent performances of corneal collagen fiber, and can be widely used in medical filed.

By repeated tests, the cornea is still transparent after being preserved in the preservation solution of the present disclosure at room temperature for 6 months; while being preserved in traditional glycerol, the cornea is subjected to slightly xanthochroia at room temperature for 1 month, and becoming moderate yellow, turning hard, lossing of original form and beingfragile at room temperature for 6 months (FIG. 2). It demonstrates that for the corneal grafts, the present preservation solution can effectively maintain its transparency, and prevent aging.

The dehydration effect of the present preservation solution can make the corneal thickness maintained to the original physiological level. A fresh porcine cornea was cut by a 500µm lamellar cornea shaper, then the thickness of the front lamella was measured by a micro thickness measuring instrument. After being divided into two eaqual pieces along the central line, one piece was put into the present preservation solution and the other into the traditional glycerol preservation solution respectively. The thickness of the corneas were measured at different times, and compared to the original thickness of the cornea, to obtain the ratios of thickness changes at different times after being put into the preservation solutions (FIG. 1). It can be observed that the traditional glycerol preservation solution has a certain dehydration effect on the cornea, which requires the cornea to be rehydrated before use in a surgery. However, the glycerol is small molecule which is easily soluble in water, and the corneal grafts may be too hard due to inadequate rehydration and thus affect operation effect since the rehydration time and degree is difficult to control. While excessive rehydration will lead to graft edema and thus affect vision recovery after surgery. The present preservation solution can effectively maintain corneal thickness, thus the corneal grafts can be sutured directly in a surgery.

Table 1 Coeneal thickness changes after the corneal graft being put into different preservation solutions (Units: mm)

**Table 2**

| Time after being put into | One week | One month | Three months |
|---|---|---|---|
| the present preservation solution | 0.95±0.27 | 0.92±0.31 | 1.05±0.29 |
| traditional glycerol preservation solution | 0.67±0.25 | 0.65±0.21 | 0.64±0.33 |

## Claims

1. A lamellar cornea preservation solution, **characterized in that** the lamellar cornea preservation solution comprises the following components:
200-500g/L of glycerol, 5-50g/L of hyaluronic acid, 10-25g/L chondroitin sulfate, 1-10g/L of an antioxidant, and 10-40mmol/L of a buffer salt solution, a pH value being 6.0-8.0.

2. The lamellar cornea preservation solution of claim 1, **characterized in that** the antioxidant is N-acetylcysteine or vitamin C.

3. The lamellar cornea preservation solution of claim 1, **characterized in that** the buffer salt solution is phosphate buffer solution, carbonate buffer solution or Hepes buffer solution.

## Patentansprüche

1. Lösung zum Konservieren lamellarer Hornhaut, **dadurch gekennzeichnet, dass** die Lösung zum Konservieren lamellarer Hornhaut die folgenden Komponenten umfasst:
200-500 g/l Glycerin, 5-50 g/l Hyaluronsäure, 10-25 g/l Chondroitinsulfat, 1-10 g/l eines Antioxidationsmittels und 10-40 mmol/l einer Puffersalzlösung, mit einem pH-Wert von 6,0-8,0.

2. Lösung zum Konservieren lamellarer Hornhaut nach Anspruch 1, **dadurch gekennzeichnet, dass** es sich bei dem Antioxidationsmittel um N-Acetylcystein oder Vitamin C handelt.

3. Lösung zum Konservieren lamellarer Hornhaut nach Anspruch 1, **dadurch gekennzeichnet, dass** es sich bei der Puffersalzlösung um Phosphatpufferlösung, Carbonatpufferlösung oder Hepes-Pufferlösung handelt.

## Revendications

1. Solution de conservation de cornée lamellaire, **caractérisée en ce que** la solution de conservation de cornée lamellaire comprend les constituants suivants :
200-500 g/L de glycérol, 5-50 g/L d'acide hyaluronique, 10-25 g/L de sulfate de chondroïtine, 1-10 g/L d'un antioxydant, et 10-40 mmol/L d'une solution saline tampon, une valeur de pH étant 6,0-8,0.

2. Solution de conservation de cornée lamellaire de la revendication 1, **caractérisée en ce que** l'antioxydant est la N-acétylcystéine ou la vitamine C.

3. Solution de conservation de cornée lamellaire de la revendication 1, **caractérisée en ce que** la solution saline tampon est une solution tampon de phosphate, une solution tampon de carbonate ou une solution tampon d'Hepes.
